# EUROPEAN PATENT APPLICATION

(11) **EP 3 960 167 A1**
(43) Date of publication of application: **02.03.2022**
(21) Application number: 19926212.2
(22) Date of filing: 05.05.2019
(51) Int. Cl.: A61K 31/197, A61P 35/00

(54) **USE OF PANTOTHENIC ACID IN PREPARATION COMPOSITION FOR TREATING AND/OR PREVENTING TUMORS**

(30) Priority: 25.04.2019 CN 201910338792
(71) Applicant: Revaissant (Shenzhen) Biosciences Co., Ltd., Guangdong 518172 (CN)
(72) Inventor: CAI, Qingqing, Shenzhen, Guangdong 518054 (CN)
(74) Representative: HGF
(86) International application number: PCT/CN2019/085472
(87) International publication number: WO 2020/215356

(57) **Abstract**

The present disclosure discloses a use of pantothenic acid in preparation of a composition for treating and/or preventing tumors. The present disclosure also discloses a use of metabolites of intestinal bacteria comprising pantothenic acid in preparation of a composition for treating and/or preventing tumors. The present disclosure also discloses a use of intestinal bacteria capable of secreting metabolites comprising pantothenic acid in preparation of a composition for treating and/or preventing tumors. Pantothenic acid and metabolites of intestinal bacteria comprising pantothenic acid can significantly promote the accumulation of CD8⁺ cytotoxic T lymphocytes in tumor microenvironment, and significantly inhibit tumor growth, thereby achieving the effect of preventing and/or treating tumors.

## Description

### TECHNICAL FIELD

The present disclosure relates to the field of biomedicine, particularly, it relates to a use of pantothenic acid in preparation of a composition for treating and/or preventing tumors, a use of metabolites of intestinal bacteria comprising pantothenic acid in preparation of a composition for treating and/or preventing tumors, and a use of intestinal bacteria capable of secreting metabolites comprising pantothenic acid in preparation of a composition for treating and/or preventing tumors.

### BACKGROUND

Malignant tumors are the top killer threatening human health and livelihood. The latest global statistics from the World Health Organization show that in 2018, there were 1.81 million new cancer cases and about 960,000 deaths from cancers. One-fifth of men and one-sixth of women in the world suffer from cancers in their lives. The incidence and mortality rates of cancers worldwide are increasing rapidly every year. In 2018, the death rate of liver cancer patients ranked fifth in the world, with 627,000 deaths, accounting for 8.2% of all cancer deaths, and both morbidity and mortality are on the rise. Radical surgical treatment is an important treatment approach for tumors. Still, most tumors, like liver cancer, are found at an advanced stage, and it is not suitable for radical surgery. Therefore, it is urgent to develop new comprehensive treatment methods, such as new targeted therapies, immunotherapy, individualized treatment and the like, and to develop safer and more effective anti-tumor drugs with less toxic side effects.

The immune system can recognize and eliminate tumor cells under normal body conditions. Still, it can be inhibited by tumor cells in various ways under pathological conditions, leading to an immune escape. Tumor immunotherapy means that the human body is actively or passively stimulated to produce tumor-specific immune responses. Thus, the activity of the immune system is recovered or enhanced in the body, and immunosuppression is relieved, and then the growth of tumor cells is inhibited. CD8⁺ cytotoxic T lymphocytes are one of the key immune cell subsets for the body to perform anti-tumor functions. At present, the effect of tumor immunotherapy is poor, mainly because the number of infiltrating CD8⁺ cytotoxic T cells is insufficient and the specificity is weak in tumor microenvironment. As a result, most patients cannot respond effectively to antibody therapeutics that blocks T cell inhibitory molecules, severely weakening the therapeutic effect. Therefore, it is urgent to develop new methods that can regulate the function of immune system in body, especially regulating the immune cells in the tumor microenvironment, such as CD8⁺ cytotoxic T cells, NK cells, macrophages, regulatory T lymphocytes (Treg), and the like.

In recent years, a number of studies have found that the human intestinal flora can regulate the immune system in many ways and play a vital role in the occurrence and development of diseases. There are about 4 to 5 million kinds of bacteria in the human intestines, most of which are anaerobic bacteria. The intestinal flora and the human body are interdependent and interact with each other. The human intestinal flora has the effect of inhibiting the growth of tumors and has broad application prospects for the prevention and treatment of tumors. However, the specific anti-tumor active substances of intestinal bacteria are not completely clear at present. The intestinal flora decomposes and utilizes nutrients in the human intestine to produce many metabolites, which could be key active small molecules that inhibit tumors. At present, there is no report that metabolites of human intestinal bacteria will enhance an accumulation of CD8⁺ cytotoxic T cells in the tumor microenvironment and have anti-tumor effects.

Pantothenic acid, also known as vitamin B₅, is a water-soluble vitamin and its molecular formula is C₉H₁₇NO₅ with a molecular weight of 219.23500. It was previously recognized that pantothenic acid was mainly involved in the synthesis of Coenzyme A (CoA), and then participated in the synthesis and metabolism of carbohydrates, fats, and proteins in the form of CoA. Pantothenic acid may have a certain protective effect on skins and mucosa, but its action mechanism is unclear. There is no report on a use of pantothenic acid for preventing and treating tumors and a use of pantothenic acid for enhancing an accumulation of CD8⁺ cytotoxic T cells in the tumor microenvironment and for preventing and treating tumors.

### SUMMARY

In order to solve the above technical problems, especially the problem of insufficient accumulation of CD8⁺ cytotoxic T lymphocytes in anti-tumor immunotherapy, an objective of the present disclosure is to provide a composition and/or a method for effectively treating and/or preventing solid tumors, in particular for enhancing an accumulation of CD8⁺ cytotoxic T lymphocytes in tumor microenvironment.

In order to achieve the above-mentioned objects, the present disclosure provides a use for treating and/or preventing tumors and a use of pantothenic acid in preparation of a composition for treating and/or preventing tumors. The use can achieve an anti-tumor effect, by enhancing an accumulation of CD8⁺cytotoxic T lymphocytes in the tumor microenvironment. Therefore, the present disclosure also provides a use of pantothenic acid in preparation of a composition for enhancing the accumulation of CD8⁺ cytotoxic T lymphocytes in the tumor microenvironment.

The pantothenic acid of the present disclosure includes but is not limited to any one of the following: stereoisomers, tautomers, geometric isomers, nitrogen oxides, hydrates, solvates, metabolites, and pharmaceutically acceptable salts or prodrugs of pantothenic acid.

The composition for treating and/or preventing tumors of the present disclosure can be, but is not limited to, a pharmaceutical composition, a food composition or a health product composition.

The tumor of the present disclosure may be various solid tumors, including but not limited to, liver cancer, breast cancer, lung cancer, melanoma, prostate cancer, fibrosarcoma, gastric cancer, esophageal cancer, colorectal cancer, bladder sarcoma, neuroglioma and the like, in particular liver and/or breast tumors.

The present disclosure also provides a use of metabolites of intestinal bacteria in preparation of a composition for treating and/or preventing tumors, wherein the metabolites of intestinal bacteria comprise pantothenic acid.

The intestinal bacteria of the present disclosure include bacteria or altered or modified bacteria comprising pantothenic acid or secreting pantothenic acid during growth. The intestinal bacteria include but are not limited to any one or more of the following: *Prevotella Copri, Akkermansia muciniphila, Bacteroides fragilis, Eubacterium, Alistipes, Clostridium, Ruminococcus, Bifidobacterium saeculare, Bacteroides stercoris, Bifidobacterium pseudocatenulatum, Butyricimonas virosa, Campylobacter ureolyticus, Enterobacter cloacae, Bacteroides ovatus, Fusobacterium varium, Bacteroides massiliensis, Bifidobacterium adolescentic, Parabacteroides goldsteinii, Sphingobacteria, and Sutterella wadsworthensis.*

The metabolite of intestinal bacteria can be, but is not limited to, any one or more of the following: culture supernate of the intestinal bacteria; culture supernate of genetically recombined, altered, modified, attenuated, chemically treated, physically treated or inactivated intestinal bacteria; and/or lysate of intestinal bacteria.

The composition for treating and/or preventing tumors can be but is not limited to a pharmaceutical composition, a food composition or a health product composition.

In another aspect, the present disclosure also provides a use of intestinal bacteria capable of secreting metabolites comprising pantothenic acid in preparation of a composition for treating and/or preventing tumors.

The metabolite can be, but is not limited to any one or more of the following: culture supernate of intestinal bacteria; culture supernate of genetically recombined, altered, modified, attenuated, chemically treated, physically treated or inactivated intestinal bacteria; and/or lysate of intestinal bacteria.

The intestinal bacteria of the present disclosure include bacteria or altered or modified bacteria comprising pantothenic acid or secreting pantothenic acid during growth. The intestinal bacteria include, but are not limited to any one or more of the following: *Prevotella Copri, Akkermansia muciniphila, Bacteroides fragilis, Eubacterium, Alistipes, Clostridium, Ruminococcus, Bifidobacterium saeculare, Bacteroides stercoris, Bifidobacterium pseudocatenulatum, Butyricimonas virosa, Campylobacter ureolyticus, Enterobacter cloacae, Bacteroides ovatus, Fusobacterium varium, Bacteroides massiliensis, Bifidobacterium adolescentic, Parabacteroides goldsteinii, Sphingobacteria,* and *Sutterella wadsworthensis.*

Preferably, the composition for treating and/or preventing tumors can be but is not limited to a pharmaceutical composition, a food composition or a health product composition.

The present disclosure also provides a use of pantothenic acid in preparation of a composition for enhancing an accumulation of CD8 positive (CD8⁺) cytotoxic T lymphocytes in tumor microenvironment.

"Enhancing the accumulation of CD8⁺ cytotoxic T lymphocytes in tumor microenvironment" includes but is not limited to a relative increase in the quantity of CD8⁺ T lymphocytes for all cells in the tumor microenvironment.

The pantothenic acid includes but is not limited to any one of the following: stereoisomers, tautomers, geometric isomers, nitrogen oxides, hydrates, solvates, metabolites, pharmaceutically acceptable salts or prodrugs of pantothenic acid.

The composition for treating and/or preventing tumors of the present disclosure can be, but is not limited to, a pharmaceutical composition, a food composition or a health product composition.

The tumor of the present disclosure can be various solid tumors, such as, but is not limited to, liver cancer, breast cancer, lung cancer, melanoma, prostate cancer, fibrosarcoma, gastric cancer, esophageal cancer, colorectal cancer, bladder sarcoma, neuroglioma and the like, especially liver and/or breast tumor.

In yet another aspect, the present disclosure also provides a use of metabolites of intestinal bacteria in preparation of a composition for enhancing an accumulation of CD8 positive (CD8⁺) cytotoxic T lymphocytes in tumor microenvironment, wherein the metabolites of intestinal bacteria comprise pantothenic acid.

In yet another aspect, the present disclosure also provides a use of intestinal bacteria capable of secreting metabolites comprising pantothenic acid in preparation of a composition for enhancing an accumulation of CD8 positive (CD8⁺) cytotoxic T lymphocytes in tumor microenvironment.

The present disclosure also provides a composition for treating and/or preventing a tumor, wherein the composition comprises pantothenic acid as an active ingredient of a drug. In certain embodiments, the composition is a pharmaceutical composition comprising pantothenic acid and a pharmaceutically acceptable carrier thereof. The tumor can be various solid tumors, such as, but is not limited to liver cancer, breast cancer, lung cancer, melanoma, prostate cancer, fibrosarcoma, gastric cancer, esophageal cancer, colorectal cancer, bladder sarcoma, neuroglioma and the like, especially can be liver and/or breast tumor.

According to one aspect of the present disclosure, in the above-mentioned pharmaceutical composition, the intestinal bacteria include bacteria or altered or modified bacteria comprising pantothenic acid or secreting pantothenic acid during growth. The intestinal bacteria include but are not limited to any one or more of the following: *Prevotella Copri, Akkermansia muciniphila, Bacteroides fragilis, Eubacterium, Alistipes, Clostridium, Ruminococcus, Bifidobacterium saeculare, Bacteroides stercoris, Bifidobacterium pseudocatenulatum, Butyricimonas virosa, Campylobacter ureolyticus, Enterobacter cloacae, Bacteroides ovatus, Fusobacterium varium, Bacteroides massiliensis, Bifidobacterium adolescentic, Parabacteroides goldsteinii, Sphingobacteria, and Sutterella wadsworthensis.*

According to one aspect of the present disclosure, in the above-mentioned pharmaceutical composition, the metabolites of intestinal bacteria include, but are not limited to, any one or more of the following: culture supernate of intestinal bacteria; culture supernate of genetically recombined, altered, modified, attenuated, chemically treated, physically treated or inactivated intestinal bacteria; and/or lysate of intestinal bacteria.

According to one aspect of the present disclosure, in the above-mentioned pharmaceutical composition, pantothenic acid can be, but is not limited to any one or more of the following: stereoisomers, tautomers, geometric isomers, nitrogen oxides, hydrates, solvates, metabolites, pharmaceutically acceptable salts or prodrugs of pantothenic acid.

According to one aspect of the present disclosure, the pharmaceutical composition of the present disclosure includes a pharmaceutically effective amount of pantothenic acid and a pharmaceutically acceptable carrier thereof, wherein pantothenic acid is an active ingredient.

Preferably, in the above-mentioned pharmaceutical composition, the pharmaceutical composition can be any one or more pharmaceutically licensed dosage forms, including but not limited to tablets, capsules, oral liquids or freeze-dried powders.

Preferably, in the above-mentioned pharmaceutical composition, the pharmaceutically acceptable carrier is one or a mixture of two or more of skimmed milk, lactose, glucose, sucrose, sorbitol, mannose, trehalose, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate or mineral oil.

The composition can also be an edible composition, which includes, but is not limited to, foods, health products, food additives, and the like.

In order to better achieve the above objective, the present disclosure also provides a composition for enhancing an accumulation of CD8⁺ cytotoxic T lymphocytes in tumor microenvironment, wherein the composition comprises pantothenic acid as an active ingredient.

In certain embodiments, the composition is a pharmaceutical composition comprising pantothenic acid and a pharmaceutically acceptable carrier thereof. The tumor can be various solid tumors, such as, but is not limited to, liver cancer, breast cancer, lung cancer, melanoma, prostate cancer, fibrosarcoma, gastric cancer, esophageal cancer, colorectal cancer, bladder sarcoma, neuroglioma and the like, especially can be liver and/or breast tumor.

According to one aspect of the present disclosure, in the above-mentioned pharmaceutical composition, the intestinal bacteria include bacteria or altered or modified bacteria comprising pantothenic acid or secreting pantothenic acid during growth. The intestinal bacteria include, but are not limited to any one or more of the following: *Prevotella Copri, Akkermansia muciniphila, Bacteroides fragilis, Eubacterium, Alistipes, Clostridium, Ruminococcus, Bifidobacterium saeculare, Bacteroides stercoris, Bifidobacterium pseudocatenulatum, Butyricimonas virosa, Campylobacter ureolyticus, Enterobacter cloacae, Bacteroides ovatus, Fusobacterium varium, Bacteroides massiliensis, Bifidobacterium adolescentic, Parabacteroides goldsteinii, Sphingobacteria, and Sutterella wadsworthensis.*

According to one aspect of the present disclosure, in the above-mentioned pharmaceutical composition, the metabolites of intestinal bacteria include, but are not limited to any one or more of the following: culture supernate of intestinal bacteria; culture supernate of genetically recombined, altered, modified, attenuated, chemically treated, physically treated or inactivated intestinal bacteria; and/or lysate of intestinal bacteria.

According to one aspect of the present disclosure, in the above-mentioned pharmaceutical composition, pantothenic acid can be, but is not limited to, any one or more of the following: stereoisomers, tautomers, geometric isomers, nitrogen oxides, hydrates, solvates, metabolites, pharmaceutically acceptable salts or prodrugs of pantothenic acid.

According to one aspect of the present disclosure, the pharmaceutical composition of the present disclosure includes a pharmaceutically effective amount of pantothenic acid and a pharmaceutically acceptable carrier thereof, wherein pantothenic acid is an active ingredient.

Preferably, in the above-mentioned pharmaceutical composition, the pharmaceutical composition can be any one or more pharmaceutically licensed dosage forms, including but not limited to tablets, capsules, oral liquids, or freeze-dried powders.

Preferably, in the above-mentioned pharmaceutical composition, the pharmaceutically acceptable carrier is one or a mixture of two or more of skimmed milk, lactose, glucose, sucrose, sorbitol, mannose, trehalose, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate or mineral oil.

The composition can also be an edible composition, which includes but is not limited to foods, health products, food additives, and the like.

The composition of the present disclosure can achieve an anti-tumor effect by enhancing an accumulation of CD8⁺ cytotoxic T lymphocytes in tumor microenvironment.

The present disclosure establishes a mouse model of liver cancer by a research method of transplantation tumor to detect and identify the effect of pantothenic acid in the mouse model of liver cancer. It is proved through experiments of the present disclosure that pantothenic acid, a metabolite of intestinal bacteria, can significantly inhibit the growth of liver tumors, and can effectively inhibit the growth of transplanted tumors in mice, suggesting that pantothenic acid, a metabolite of intestinal bacteria, has significant development and practical values in the clinical treatment for tumors.

### BRIEF DESCRIPTION OF DRAWINGS

Figure 1 is a schematic diagram of an experimental procedure of detecting intestinal bacteria *(Prevotella Copri* as an example) for treating a tumor in a mouse model of liver cancer. Time is expressed in days (Day, d) for administrating intestinal bacteria *(Prevotella Copri* as an example) or for transplanting tumor cells.
Figure 2 shows a statistical graph of relative quantification of pantothenic acid in the screening test of metabolites of intestinal bacteria.
Figure 3 is a schematic diagram of an experimental procedure for detecting pantothenic acid, the metabolite of intestinal bacteria to enhance an accumulation of CD8⁺ cytotoxic T lymphocytes in the tumor microenvironment and anti-tumor effect in a mouse model of liver cancer. Time is expressed in days (Day, d) for administering pantothenic acid or for transplanting tumor cells.
Figure 4 is a comparison diagram of tumor sizes of liver cancer cell transplanted tumors in three typical mice in each group after administration of pantothenic acid for treatment.
Figure 5 is a statistical analysis diagram of the comparison diagram of tumor sizes of liver cancer cell transplanted tumors in nine typical mice in each group after administration of pantothenic acid for treatment.
Figure 6 is an analysis diagram of flow cytometric of CD8⁺ T lymphocytes in one typical mouse in each group after pantothenic acid is administrated to mice transplanted with liver cancer cells, wherein right lower quadrant indicates CD8⁺ T lymphocytes, and numerics in the right lower quadrant show the percentage of CD8⁺ T lymphocytes in total cells in the liver tumor microenvironment.
Figure 7 is a statistical analysis diagram of the percentage of CD8⁺ T lymphocytes in total cells in the tumor microenvironment after pantothenic acid is administrated to the mice transplanted with liver cancer cells.

### DETAILED DESCRIPTION

The present disclosure will be further described below with reference to the embodiments. It should be pointed out that pantothenic acid, the metabolite of the intestinal bacteria for preventing and treating tumors in the present disclosure, or the pharmaceutical compositions, food, health products and food additives comprising pantothenic acid, the metabolite of intestinal bacteria of the present disclosure, can be applied to the indications described above and exhibits the functions described above, after being administrated to a test subject. All dosage forms within the scope of the present disclosure have been tested, and only small parts of them are described hereinafter in the examples for illustration, however, they should not be understood as a limitation of the present disclosure.

The intestinal bacteria indicated in the present disclosure include bacteria or altered or modified bacteria comprising pantothenic acid or secreting pantothenic acid during growth. The intestinal bacteria include, but are not limited to any one of the following: *Prevotella Copri, Akkermansia muciniphila, Bacteroides fragilis, Eubacterium, Alistipes, Clostridium, Ruminococcus, Bifidobacterium saeculare, Bacteroides stercoris, Bifidobacterium pseudocatenulatum, Butyricimonas virosa, Campylobacter ureolyticus, Enterobacter cloacae, Bacteroides ovatus, Fusobacterium varium, Bacteroides massiliensis, Bifidobacterium adolescentic, Parabacteroides goldsteinii, Sphingobacteria, Sutterella wadsworthensis and the like.* In certain embodiments, the intestinal bacteria include, but are not limited to *Prevotella Copri.*

The "metabolite of intestinal bacteria" indicated in the present disclosure refers to a gut microbiota-derived metabolite (such as human intestinal bacteria), and includes but is not limited to any one or more of the following: culture supernate of intestinal bacteria; culture supernate of genetically recombined, altered, modified, attenuated, chemically treated, physically treated or inactivated intestinal bacteria; and/or lysate of intestinal bacteria.

The "pantothenic acid", the metabolite of intestinal bacteria indicated in the present disclosure includes but is not limited to any of the following: stereoisomers, tautomers, geometric isomers, nitrogen oxides, hydrates, solvents of pantothenic acid

The tumor is a solid tumor, such as, but is not limited to, liver cancer, breast cancer, melanoma, lung cancer, prostate cancer, fibrosarcoma, gastric cancer, esophageal cancer, bladder sarcoma, neuroglioma and the like. In certain embodiments, the tumor includes, but is not limited to liver cancer.

The present disclosure also provides a pharmaceutical composition for treating and/or preventing tumors, which includes a pharmacologically effective amount of pantothenic acid, the metabolite of intestinal bacteria, wherein the so-called "pharmaceutically effective amount" is 0.01~0.1g/ml, preferably 0.01~0.02g/ml, 0.01~0.03g/ml, 0.01~0.04g/ml, 0.01~0.05g/ml, 0.01~0.06g/ml, 0.01~0.07g/ml, 0.01~0.08g/ml, 0.01~0.09g/ml, most preferably 0.02g/ml.

The pharmaceutical composition can be a variety of dosage forms, including but not limited to tablets, capsules, oral liquids or freeze-dried powders.

The pharmaceutically acceptable carrier includes, but is not limited to one or more of skimmed milk, lactose, glucose, sucrose, sorbitol, acacia gum, mannose, starch, trehalose, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate or mineral oil.

The "pantothenic acid", the metabolite of intestinal bacteria of the present disclosure can also be made into foods, health products or food additives, and so on. The foods, health products or food additives all contain any one or more of stereoisomers, tautomers, geometric isomers, nitrogen oxides, hydrates, solvates, metabolites, pharmaceutically acceptable salts or prodrugs of pantothenic acid. These foods, health products or food additives all can treat and/or prevent tumors.

### Example 1 An Experiment of Intestinal Bacteria for Tumor Treatment

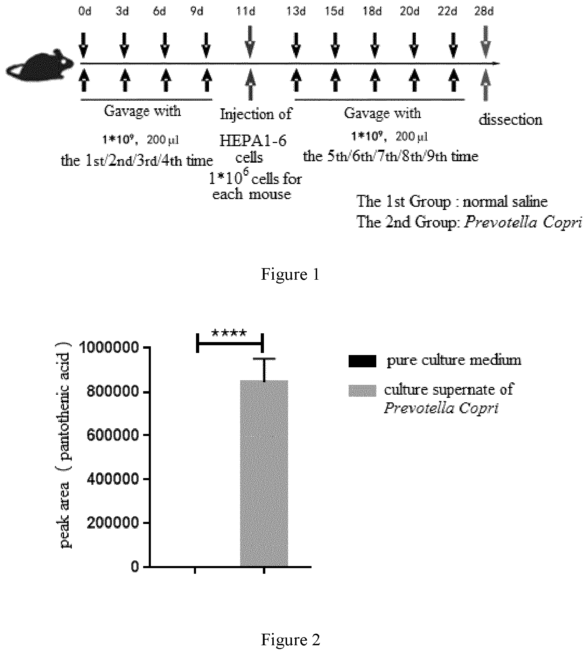

Figure 1 is a schematic diagram of an experimental procedure of detecting intestinal bacteria for treating a tumor.

### 1. Culture of Intestinal Bacteria

*Prevotella Copri* was taken as an example in this embodiment, but the technical solution of the present disclosure was not limited to *Prevotella Copri,* and also included the following intestinal bacteria: *Akkermansia muciniphila, Bacteroides fragilis, Eubacterium, Alistipes, Clostridium, Ruminococcus, Bifidobacterium saeculare, Bacteroides stercoris, Bifidobacterium pseudocatenulatum, Butyricimonas virosa, Campylobacter ureolyticus, Enterobacter cloacae, Bacteroides ovatus, Fusobacterium varium, Bacteroides massiliensis, Bifidobacterium adolescentic, Parabacteroides goldsteinii, Sphingobacteria, Sutterella wadsworthensis, and the like.* These intestinal bacteria all have characteristic of comprising pantothenic acid or secreting pantothenic acid during their growth in common.
Step 1: A lyophilized preserved *Prevotella copri* (purchased from the ATCC official website) was taken, and then 200 microliters of PYG culture medium was added to redissolve it. 20 microliters of bacterial solution was pipetted and streaked on a blood agar plate. After an air exhaustion processing by an anaerobic jar gassing system, the agar plate was placed in a biochemical incubator and cultured anaerobically at 37°C for 48 hours;
Step 2: A monoclonal colony was selected to inoculate in 10mL PYG culture medium, and cultured anaerobically at 37°C for 12 hours;
Step 3: 1% (v/v) of strains were inoculated in 500mL PYG culture medium in a flask and cultured anaerobically at 37°C for 48 hours.
Step 4: The bacterial solution was collected and centrifuged at 6000 rpm for 10 min. Culture supernate was taken after centrifugation and frozen at - 80 degrees Celsius for storage. The bacterial sludge was washed twice with saline, and was finally redissolved with saline for later use. Then the viable bacteria were counted.

### 2. An experiment of anti-tumor effect of intestinal bacteria in mice

*Prevotella Copri* was taken as an example in this embodiment, but this embodiment is not limited to *Prevotella Copri,* and also includes the following intestinal bacteria: *Akkermansia muciniphila, Bacteroides fragilis, Eubacterium, Alistipes, Clostridium, Ruminococcus, Bifidobacterium saeculare, Bacteroides stercoris, Bifidobacterium pseudocatenulatum, Butyricimonas virosa, Campylobacter ureolyticus, Enterobacter cloacae, Bacteroides ovatus, Fusobacterium varium, Bacteroides massiliensis, Bifidobacterium adolescentic, Parabacteroides goldsteinii, Sphingobacteria, Sutterella wadsworthensis,* and the like.

Experimental animals: Eighteen C57BL/6 mice aged 3 to 4 weeks old in good mental state were purchased from the Experimental Animal Center of Sun Yat-sen University. The mice were randomly divided into 2 groups, nine mice in each group, and the 2 groups were a control group and a live bacteria gavage group respectively. The 2 groups of mice were continually treated by gavage with normal saline and *Prevotella Copri* (10⁹ CFU) for 4 times, respectively. After the mouse tumor (liver cancer) cells HEPA1-6 grew at logarithmic phase, they were digested with trypsin, and culture medium was neutralized. After centrifugation, the cells were collected, washed twice with DPBS to remove residual serum. Finally, the cells were resuspended with DPBS. When the cell counting was completed, each mouse was subcutaneously inoculated with 10⁶ cells into right axilla and continually treated by gavage (gavage with 1 × 10⁹ CFU, 200µl, in the 5^{th}/6^{th}/7^{th}/8^{th}/9^{th} time), and the tumor-bearing mice were killed after 2 weeks. The sizes of the tumors in mice were recorded to calculate tumor inhibition rates, respectively.

The experimental procedure of administrating intestinal bacteria for treating tumors in mice transplanted with liver cancer cell was shown in Figure 1, wherein *Prevotella Copri* was taken as an example, but the experiment was not limited to *Prevotella Copri.* Time was expressed in days (Day, d) for administrating intestinal bacteria *(Prevotella Copri* as an example) or for transplanting tumor cells.

The results showed that the sizes of the mouse tumors were significantly reduced by about 40-88% after the administration of *Prevotella Copri,* compared with the normal saline control group, and the reduction represents statistical difference (*p*<0.05).

In other embodiments (the conditions were all the same, except for different bacterial species), results showed that the following intestinal bacteria all had obvious effects on inhibiting tumor growth: *Akkermansia muciniphila* (tumor inhibition rate: about40%~80%), *Bacteroides fragilis* (tumor inhibition rate: about 40%~85%), *Eubacterium* (tumor inhibition rate: about 31%~66%), *Alistipes* (tumor inhibition rate: about 27%~63%), *Clostridium* (tumor inhibition rate: about 29%~78%), *Ruminococcus* (tumor inhibition rate: about 35%~78%), *Bifidobacterium saeculare* (tumor inhibition rate: about 28%~71%), *Bacteroides stercoris* (tumor inhibition rate: about 33%~62%), *Bifidobacterium pseudocatenulatum* (tumor inhibition rate: about 26%~56%), *Butyricimonas virosa* (tumor inhibition rate: about 31%~53%), *Campylobacter ureolyticus* (tumor inhibition rate: about 30%~62%), *Enterobacter cloacae* (tumor inhibition rate: about 26%~71%), *Bacteroides ovatus* (tumor inhibition rate: about 35%~51%), *Fusobacterium varium* (tumor inhibition rate: about 37%~56%), *Bacteroides massiliensis* (tumor inhibition rate: about 34%~58%), *Bifidobacterium adolescentic* (tumor inhibition rate: about 30%~55%), *Parabacteroides goldsteinii* (tumor inhibition rate: about 28%~75%), *Sphingobacteria* (tumor inhibition rate: about 27%~74%), *Sutterella wadsworthensis* (tumor inhibition rate: about 22%~55%).

### Example 2 A Screening Experiment of Dominant Metabolites of Intestinal Bacteria

### 1. Culture Supernate of Intestinal bacteria

*Prevotella Copri* was taken as an example in this embodiment, but the technical solution of the present disclosure is not limited to *Prevotella Copri,* and also includes the following Intestinal Bacteria: *Akkermansia muciniphila, Bacteroides fragilis, Eubacterium, Alistipes, Clostridium, Ruminococcus, Bifidobacterium saeculare, Bacteroides stercoris, Bifidobacterium pseudocatenulatum, Butyricimonas virosa, Campylobacter ureolyticus, Enterobacter cloacae, Bacteroides ovatus, Fusobacterium varium, Bacteroides massiliensis, Bifidobacterium adolescentic, Parabacteroides goldsteinii, Sphingobacteria, Sutterella wadsworthensis,* and the like.
Step 1: A lyophilized preserved *Prevotella copri* (purchased from the ATCC official website) was taken, and then 200 microliters of PYG culture medium was added to redissolve it. 20 microliters of bacterial solution was pipetted and streaked on a blood agar plate. After an air exhaustion processing by an anaerobic jar gassing system, the agar plate was placed in a biochemical incubator and cultured anaerobically at 37°C for 48 hours;
Step 2: A monoclonal colony was selected to inoculate in 10mL PYG culture medium, and cultured anaerobically at 37°C for 12 hours;
Step 3: 1% (v/v) of strains were inoculated in 500mL PYG culture medium in a flask and cultured anaerobically at 37°C for 48 hours.
Step 4: The bacterial solution was collected and centrifuged at 6000 rpm for 10 min. Culture supernate was taken after centrifugation and frozen at - 80 degrees Celsius for storage. The bacterial sludge was washed twice with normal saline, and was finally redissolved with normal saline for later use. Then the viable bacteria were counted and the concentration of the supernate was quantified.

### 2. A Screening Metabolite Experiment of Culture Supernate of Intestinal Bacteria

*Prevotella Copri* was taken as an example in this embodiment, but the technical solution of the present disclosure is not limited to *Prevotella Copri,* and also includes the following Intestinal Bacteria: *Akkermansia muciniphila, Bacteroides fragilis, Eubacterium, Alistipes, Clostridium, Ruminococcus, Bifidobacterium saeculare, Bacteroides stercoris, Bifidobacterium pseudocatenulatum, Butyricimonas virosa, Campylobacter ureolyticus, Enterobacter cloacae, Bacteroides ovatus, Fusobacterium varium, Bacteroides massiliensis, Bifidobacterium adolescentic, Parabacteroides goldsteinii, Sphingobacteria, Sutterella wadsworthensis,* and the like.

Culture supernate of Intestinal bacteria was divided into a pure culture medium (PYG medium) group as a control group and a culture supernate group of *Prevotella copri* as an experimental group, with 6 replicates in each group.
1. 200 microliters of sample was taken into a 1.5 ml EP tube, and 300 microliters of extracting solution (methanol) was added, then 10 microliters of ribitol (dissolved in ultrapure water, 1mg/mL) was added, and the solution was then vortex mixed for 30s;
2. The solution was centrifuged at 12000rpm at 4°C for 15min;
3. 350 microliters of the supernate was carefully pipetted into a 2 ml vial (silanized);
4. A vacuum concentrator was used for drying an extract;
5. 40 microliters of methoxyamine salt (methoxyamine hydrochloride, dissolved in pyridine, 20mg/mL) was added to the dried metabolites, mixed gently, and the mixture was incubated in an oven at 80°C for 30 minutes;
6.60 microliters of BSTFA (containing 1% TMCS, v/v) was added to each sample, and the mixture was incubated at 70°C for 1.5 hours;
7. The sample was tested with Agilent 7890 gas chromatography-time-of-flight mass spectrometer (GC-TOFMS);
8. Mass spectrum data were analyzed by Chroma TOF software (V 4.3x, LECO) and a series of analyses, such as peak extraction, baseline correction, deconvolution, peak integration, peak alignment and the like were performed. The LECO-Fiehn Rtx5 database was used to perform a qualitative analysis, including mass spectrometry matching and retention time index matching.
9. The types of metabolites and peak areas between the two groups were statistically analyzed.

The results were shown in Figure 2. It was found that a relative quantity of pantothenic acid in the *Prevotella Copri* supernate was significantly increased, and the increase represents a statistically significant difference (*p*<0.0001), by screening and analyzing the types of metabolites and peak areas in the culture supernate of *Prevotella Copri* with gas chromatography-time-of-flight mass spectrometry (GC-TOFMS).

In other embodiments (the conditions were all the same, except for different bacterial species), results showed that, compared with the pure culture medium group, the relative quantities of pantothenic acid in the experimental group were all significantly increased during culture of the following intestinal bacteria: *Akkermansia muciniphila* (peak area increased by about 80%), *Bacteroides fragilis* (peak area increased by about 90%), *Eubacterium* (peak area increased by about 81%), *Alistipes* (peak area increased by about 75%), *Clostridium* (peak area increased by about 65%), *Ruminococcus* (peak area increased by about 68%), *Bifidobacterium saeculare* (peak area increased by about 65%), *Bacteroides stercoris* (peak area increased by about 72%), *Bifidobacterium pseudocatenulatum* (peak area increased by about 52%), *Butyricimonas virosa* (peak area increased by about 51%), *Campylobacter ureolyticus* (peak area increased by about 75%), *Enterobacter cloacae* (peak area increased by about 58%), *Bacteroides ovatus* (peak area increased by about 85%), *Fusobacterium varium* (peak area increased by about 82%), *Bacteroides massiliensis* (peak area increased by about 76%), *Bifidobacterium adolescentic* (peak area increased by about *74%), Parabacteroides goldsteinii* (peak area increased by about 68%), *Sphingobacteria* (peak area increased by about 80%), *Sutterella wadsworthensis* (peak area increased by about 60%).

The amount of pantothenic acid obviously increases during the culture of above-mentioned bacterial, indicating that the intestinal bacteria produce and secrete a large amount of pantothenic acid, which is one of the key active small molecular substances for the intestinal bacteria to inhibit tumors.

### Example 3 An Experiment of Pantothenic Acid in Enhancing an Accumulation of CD8⁺ Cytotoxic T Lymphocytes and an Anti-Tumor Effect in the Tumor Microenvironment

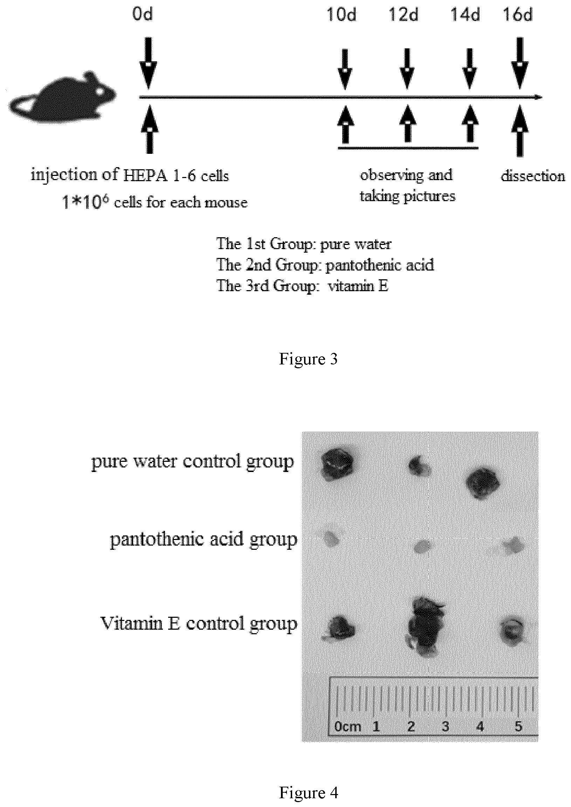

Figure 3 is a schematic diagram of the experimental procedure for detecting pantothenic acid, the metabolite of the intestinal bacteria to enhance an accumulation of CD8⁺ cytotoxic T cells in the tumor microenvironment and to treat tumors in a mouse model of liver cancer. That is, an experimental procedure of administering pantothenic acid to mice subcutaneously transplanted with liver cancer cells (HEPA1-6). The time is expressed in days (Day, d) for administering pantothenic acid or for transplanting tumor cells.

### An Experiment of Pantothenic Acid for Tumor Prevention and Treatment in Mice

Experimental animals: Twenty-seven C57BL/6 mice aged 3 to 4 weeks old in good mental state were purchased from the Experimental Animal Center of Sun Yat-sen University. The mice were randomly divided into 3 groups, nine mice for each group, and the 3 groups were a control group, a pantothenic acid group and a Vitamin E control group respectively. The 3 groups of mice were continually administered by gavage with pure water, pantothenic acid (at a concentration of 0.02g/ml, purchased from the Sigma official website) and Vitamin E (at a concentration of 0.02g/ml, purchased from the Sigma official website) for 3 to 4 weeks, respectively. After the mouse tumor (liver cancer) cells HEPA1-6 grew at logarithmic phase, they were digested with trypsin, and culture medium was neutralized. After centrifugation, the cells were collected, washed twice with DPBS to remove residual serum. Finally, the cells were redissolved with DPBS. After cell counting was completed, each mouse was subcutaneously inoculated with 10⁶ cells into right axilla and continually treated by gavage, respectively, and the tumor-bearing mice were killed after 2 to 3 weeks. After dissection, the sizes of the subcutaneously transplanted tumors were measured, and the tumor cells were collected in situ. A relative amount of the CD8⁺ T lymphocytes in the tumor microenvironment was detected and analyzed by flow cytometry.

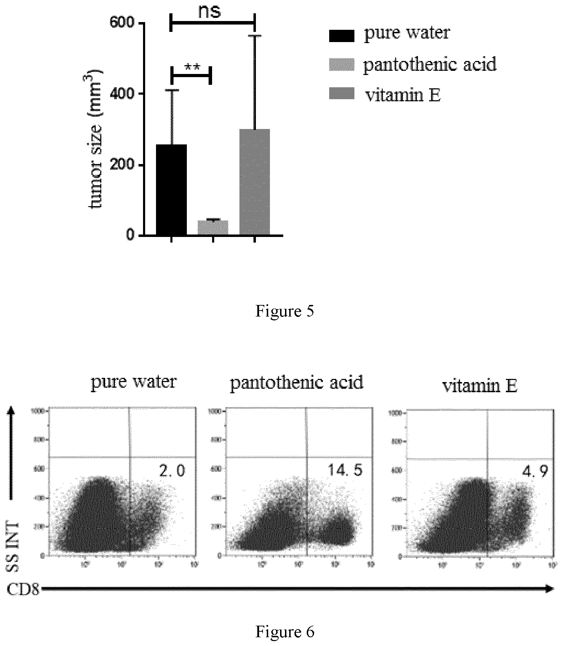

Figure 4 is a comparison diagram of tumor size of three typical mice transplanted with liver cancer cell in each group, after an administration of pantothenic acid. Figure 5 is a statistical analysis diagram of the comparison diagram of tumor size of nine typical mice transplanted with liver cancer cell in each group, after an administration of pantothenic acid. From the experimental results of Figure 4 and Figure 5, it can be clearly observed that after the administration of pantothenic acid, the size of liver cancer cell transplanted tumors are greatly reduced by about 80%, and the reduction represents a statistically significant difference (*p*<0.01). It can be concluded that the treatment of administrating pantothenic acid can significantly inhibit the tumor growth.

Figure 6 is an analysis diagram of flow cytometric of the amount of CD8⁺ T lymphocytes in one typical mouse transplanted with liver cancer cell in each group in tumor microenvironment, after an administration of pantothenic acid, wherein right lower quadrants indicates CD8⁺ T lymphocytes, and numerics in the right lower quadrants show the percentage of CD8⁺ T lymphocytes in total cells in the liver tumor microenvironment. According to the diagram of flow cytometry, compared with the Vitamin E control group, pantothenic acid increases the relative amount of CD8⁺ T lymphocytes in the liver tumor microenvironment by more than 3 times. Compared with the pure water control group, pantothenic acid increases the relative amount of CD8⁺ T lymphocytes by more than 7 times.

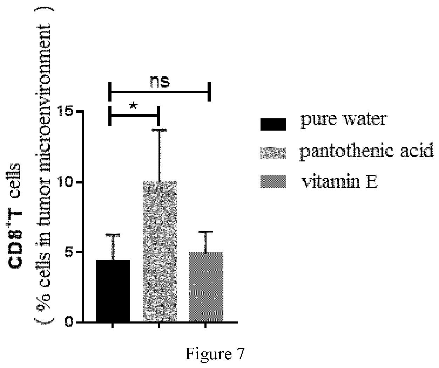

Figure 7 is a statistical analysis diagram of the percentages of CD8⁺ T lymphocytes in total cells in tumor microenvironment, after an administration of pantothenic acid for the mice transplanted with liver cancer cells. It can be seen from the statistical diagram that, compared with the pure water control group and the Vitamin E control group, pantothenic acid significantly increases the relative amount of CD8⁺ T lymphocytes in the tumor microenvironment, and the increase represents a statistically significant difference (*p*<0.05).

In the statistical analysis diagram of the above results, ns indicates non statistically significant difference, ^{∗} indicates the student t-test at *p*<0.05, ^{∗∗} indicates the student t-test at *p*<0.01, and ^{∗∗∗∗} indicates the student t-test at *p*<0.0001. *p<0.05* means a statistically significant difference. In the experiment of supernate, there were 6 replicates in each group, and in the animal experiment, there were 9 mice in each group.

In summary, intestinal bacteria have a significant inhibitory effect on tumor growth, wherein pantothenic acid, the metabolite of intestinal bacteria is one of the key active small molecular substances to play an inhibitory effect (Figure 2). Pantothenic acid is capable of significantly inhibiting the formation and growth of mouse tumors (liver cancer cell transplanted tumors) (Figure 4 and Figure 5). In addition, it can be seen from the experimental results of Figure 7 and Figure 8 that in the mouse tumors (liver cancer cell transplanted tumors) of the experimental group, pantothenic acid promotes the accumulation of CD8⁺ T lymphocytes in the tumor microenvironment *in vivo,* thereby inhibiting tumor growth and effectively treating and/or preventing tumors (especially liver tumors).

The above content is a further detailed description of the technical solution in combination with the preferred embodiments of the present disclosure. It cannot be considered that the specific implementation of the present disclosure is limited to the above descriptions. For those skilled in the art, any simple deductions or replacements without departing from the inventive concept of the disclosure should all be regarded as belonging to the protection scope of the disclosure.

## Claims

1. A use of pantothenic acid in preparation of a composition for treating and/or preventing tumors.

2. The use according to claim 1, **characterized in that** the pantothenic acid is any one or more of the following: stereoisomers, tautomers, geometric isomers, nitrogen oxides, hydrates, solvates, metabolites, and pharmaceutically acceptable salts or prodrugs of pantothenic acid.

3. The use according to claim 1, **characterized in that** the composition is a pharmaceutical composition or a food composition or a health product composition.

4. The use according to claim 1, **characterized in that** the tumor is a solid tumor.

5. The use according to claim 1, **characterized in that** the tumor is a liver tumor.

6. A use of a metabolite of intestinal bacteria in preparation of a composition for treating and/or preventing tumors, **characterized in that** the metabolite of intestinal bacterial comprises pantothenic acid.

7. The use according to claim 6, **characterized in that** the metabolite of intestinal bacteria is any one or more of the following: culture supernate of intestinal bacteria; culture supernate of genetically recombined, altered, modified, attenuated, chemically treated, physically treated or inactivated intestinal bacteria; and/or lysate of intestinal bacteria.

8. The use according to claim 6, **characterized in that** the intestinal bacteria include bacteria or altered or modified bacteria comprising pantothenic acid or secreting pantothenic acid during growth.

9. The use according to claim 6, **characterized in that** the intestinal bacteria include any one or more of *Prevotella Copri, Akkermansia muciniphila, Bacteroides fragilis, Eubacterium, Alistipes, Clostridium, Ruminococcus, Bifidobacterium saeculare, Bacteroides stercoris, Bifidobacterium pseudocatenulatum, Butyricimonas virosa, Campylobacter ureolyticus, Enterobacter cloacae, Bacteroides ovatus, Fusobacterium varium, Bacteroides massiliensis, Bifidobacterium adolescentic, Parabacteroides goldsteinii, Sphingobacteria, and Sutterella wadsworthensis.*

10. The use according to claim 6, **characterized in that** the composition is a pharmaceutical composition, a food composition or a health product composition.

11. A use of intestinal bacteria capable of secreting a metabolite comprising pantothenic acid in preparation of a composition for treating and/or preventing tumors.

12. The use according to claim 11, **characterized in that** the metabolite is any one or more of the following: culture supernate of intestinal bacteria; culture supernate of genetically recombined, altered, modified, attenuated, chemically treated, physically treated or inactivated intestinal bacteria; and/or lysate of intestinal bacteria.

13. The use according to claim 11, **characterized in that** the intestinal bacteria include bacteria or altered or modified bacteria comprising pantothenic acid or secreting pantothenic acid during growth.

14. The use according to claim 11, **characterized in that** the intestinal bacteria include any one or more of *Prevotella Copri, Akkermansia muciniphila, Bacteroides fragilis, Eubacterium, Alistipes, Clostridium, Ruminococcus, Bifidobacterium saeculare, Bacteroides stercoris, Bifidobacterium pseudocatenulatum, Butyricimonas virosa, Campylobacter ureolyticus, Enterobacter cloacae, Bacteroides ovatus, Fusobacterium varium, Bacteroides massiliensis, Bifidobacterium adolescentic, Parabacteroides goldsteinii, Sphingobacteria, and Sutterella wadsworthensis.*

15. The use according to claim 11, **characterized in that** the composition is a pharmaceutical composition, or a food composition, or a health product composition.

16. A use of pantothenic acid in preparation of a composition for enhancing an accumulation of CD8⁺ cytotoxic T lymphocytes in tumor microenvironment.

17. The use according to claim 16, **characterized in that** the pantothenic acid is any one or more of the following: stereoisomers, tautomers, geometric isomers, nitrogen oxides, hydrates, solvates, metabolites, pharmaceutically acceptable salts or prodrugs of pantothenic acid.

18. The use according to claim 16, **characterized in that** the composition is a pharmaceutical composition, a food composition, or a health product composition.

19. The use according to claim 16, **characterized in that** the tumor is a solid tumor.

20. The use according to claim 16, **characterized in that** the tumor is a liver tumor.

21. A use of a metabolite of intestinal bacteria in preparation of a composition for enhancing an accumulation of CD8 positive (CD8⁺) cytotoxic T lymphocytes in tumor microenvironment, **characterized in that** the metabolite of intestinal bacteria comprises pantothenic acid.

22. A use of intestinal bacteria capable of secreting metabolite comprising pantothenic acid in preparation of a composition for enhancing an accumulation of CD8⁺ cytotoxic T lymphocytes in tumor microenvironment.

23. A composition for treating and/or preventing a tumor, wherein the composition comprises pantothenic acid as an active ingredient.

24. The composition according to claim 23, **characterized in that** the composition is a pharmaceutical composition which comprises pantothenic acid and a pharmaceutically acceptable carrier thereof.

25. A composition for enhancing an accumulation of CD8⁺ cytotoxic T lymphocytes in tumor microenvironment, **characterized in that** the composition comprises pantothenic acid as an active ingredient.

26. The composition according to claim 25, **characterized in that** the composition is a pharmaceutical composition which comprises pantothenic acid and a pharmaceutically acceptable carrier thereof.
